# EUROPEAN PATENT SPECIFICATION

(11) **EP 4 101 459 B1**
(45) Date of publication and mention of the grant of the patent: **30.07.2025**
(21) Application number: 22177062.1
(22) Date of filing: 02.06.2022
(51) Int. Cl.: A61K 36/53, A61P 25/28

(54) **COMPOSITION FOR PREVENTING, AMELIORATING, OR TREATING ALZHEIMER'S DISEASE CONTAINING BASIL EXTRACT AS ACTIVE INGREDIENT**
ZUSAMMENSETZUNG ZUM VORBEUGEN, LINDERN ODER BEHANDELN VON MORBUS ALZHEIMER, DIE BASILIKUM-EXTRAKT ALS WIRKSTOFF ENTHÄLT
COMPOSITION DESTINÉ À LA PRÉVENTION, À L'AMÉLIORATION OU AU TRAITEMENT DE LA MALADIE D'ALZHEIMER CONTENANT UN EXTRAIT DE BASILIC EN TANT QUE PRINCIP ACTIF

(30) Priority: 10.06.2021 KR 20210075285
(43) Date of publication of application: 14.12.2022
(73) Proprietor: Korea Institute of Science and Technology, Seoul 02792 (KR)
(72) Inventor: KIM, Ho Youn, 25451 Gangwon-do (KR); RYU, Da Hye, 25451 Gangwon-do (KR); NHO, Chu Won, 25451 Gangwon-do (KR); YANG, Seung Hoon, 02815 Seoul (KR)
(74) Representative: advotec.

(56) References cited:
- JP-A- 2010 202 606
- US-A1- 2014 322 198
- GRADINARIU VERONICA ET AL: "Comparative efficacy ofOcimum sanctum L.andOcimum basilicum L.essential oils against amyloid beta (1-42)-induced anxiety and depression in laboratory rats", PHYTOCHEMISTRY REVIEWS, KLUWER, NL, vol. 14, no. 4, 28 November 2014 (2014-11-28), pages 567 - 575, XP035521514, ISSN: 1568-7767, [retrieved on 20141128], DOI: 10.1007/S11101-014-9389-6
- RAZAZZAN ATEFEH ET AL: "Activation of Microbiota Sensor- Free Fatty Acid Receptor 2 Signaling Ameliorates Amyloid-[Beta] Induced Neurotoxicity by Modulating Proteolysis/Senescence Axis", 24 December 2020 (2020-12-24), XP055960979, Retrieved from the Internet <URL:https://www.preprints.org/manuscript/202012.0635/v1> [retrieved on 20220914], DOI: 10.20944/preprints202012.0635.v1
- AYE AYE ET AL: "Anti-inflammatory activity of ethanol extract of leaf and leaf callus of basil (Ocimum basilicumL.) on RAW 264.7 macrophage cells", ORIENTAL PHARMACY AND EXPERIMENTAL MEDICINE, KYUNG HEE ORIENTAL MEDICINE RESEARCH CENTER, KYUNG HEE UNIVERSITY, KR, vol. 19, no. 2, 27 April 2019 (2019-04-27), pages 217 - 226, XP036795406, ISSN: 1598-2386, [retrieved on 20190427], DOI: 10.1007/S13596-019-00372-2
- HESHAMI NEDA ET AL: "Favorable effects of dill tablets and Ocimum basilicum L. extract on learning, memory, and hippocampal fatty acid composition in hypercholesterolemic rats", IRANIAN JOURNAL OF BASIC MEDICAL SCIENCES, 1 March 2021 (2021-03-01), Iran, pages 300 - 311, XP055961002, Retrieved from the Internet <URL:https://www.ncbi.nlm.nih.gov/pmc/articles/PMC8087851/> [retrieved on 20220914], DOI: 10.22038/ijbms.2021.49013.11230
- AYUOB NASRA NAEIM ET AL: "Ocimum basilicumimprove chronic stress-induced neurodegenerative changes in mice hippocampus", METABOLIC BRAIN DISEASE, KLUWER ACADEMIC - PLENUM PUBLISHERS, NEW YORK, NY, US, vol. 33, no. 3, 22 January 2018 (2018-01-22), pages 795 - 804, XP036505959, ISSN: 0885-7490, [retrieved on 20180122], DOI: 10.1007/S11011-017-0173-3
- VENANCIO A M ET AL: "Chemical composition, acute toxicity, and antinociceptive activity of the essential oil of a plant breeding cultivar of basil (Ocimum basilicum L.)", PLANTA MEDICA, THIEME VERLAG, DE, 1 June 2011 (2011-06-01), XP018501822, ISSN: 0032-0943, DOI: 10.1055/S-0030-1250607
- MERLINI GIAMPAOLO ET AL: "Amyloidosis: Pathogenesis and New Therapeutic Options", JOURNAL OF CLINICAL ONCOLOGY, vol. 29, no. 14, 10 May 2011 (2011-05-10), US, pages 1924 - 1933, XP055961029, ISSN: 0732-183X, DOI: 10.1200/JCO.2010.32.2271
- HASE TOMOKI ET AL: "Rosmarinic acid suppresses Alzheimer's disease development by reducing amyloid [beta] aggregation by increasing monoamine secretion", SCIENTIFIC REPORTS, vol. 9, no. 1, 18 June 2019 (2019-06-18), XP093064901, Retrieved from the Internet <URL:https://www.nature.com/articles/s41598-019-45168-1> DOI: 10.1038/s41598-019-45168-1

## Description

### BACKGROUND

### (a) Technical Field

The present invention relates to a composition for preventing, ameliorating, or treating tauopathy characterized by Alzheimer's disease containing a basil extract as an active ingredient.

### (b) Background Art

Dementia is a neurodegenerative disease characterized by remarkably reduced cognitive function due to brain atrophy, cerebrovascular disorders, or the like. Dementia is classified into several types depending on the cause thereof, with Alzheimer's type dementia (AD) accounting for 60% to 80% of all dementia patients. The mechanism of onset of AD is complicated, but is considered to be caused by the formation of senile plaques attributable to the aggregation of amyloid β (Aβ) protein or variation in neurofibrillary tangles attributable to the aggregation of phosphorylated tau protein (p-tau). The number of AD patients is increasing all over the world, including in Korea. Therefore, treatment of AD is important for preventing deterioration in the patient's quality of life, reducing the burden on the family, and reducing medical expenses for the aging society of the future.

The dementia symptoms include main symptoms centered on cognitive dysfunction and secondary symptoms such as behavioral problems observed in social relationships among patients exhibiting cognitive dysfunction. Therapeutic agents for AD currently used in Japan include four types, namely donepezil hydrochloride, galantamine hydrobromide, and rivastigmine as acetylcholinesterase inhibitors and memantine hydrochloride, as an antagonist of N-methyl-D-aspartate receptor, all of which can alleviate main symptoms or secondary symptoms. However, these drugs are merely symptomatic therapies for ameliorating main symptoms or secondary symptoms for a certain period of time, and have no activity of suppressing neurodegeneration of AD. Although these drugs show a temporary cognitive function improvement effect at the beginning of use, the cognitive function generally deteriorates after 48 weeks or more, compared to the cognitive function before treatment.

It is known that the amount of Aβ, which is considered to be the cause of AD onset, is controlled based on production thereof by cleavage of precursor proteins and removal thereof by glial cells in the brain, and Aβ accumulates in the form of soluble oligomers and insoluble aggregates in the brain with age. Soluble Aβ in the brain is introduced by astrocytes and microglia. Meanwhile, Aβ that has become insoluble and aggregated is phagocytosed by microglia expressing complement receptors and IgG receptors, and is excreted into the cerebrospinal fluid (CSF), lymph, or blood. Aβ in CSF decreases with the progression of AD. This is considered to suggest an increase in aggregate Aβ in the brain. In addition, the literature relating to the diagnostic criteria for AD discloses a decrease in the amount of Aβ in CSF and an increase in the accumulation of amyloid tracers in PET images as biomarkers of Aβ accumulation in the brain.

It is known that an increase in insoluble Aβ in the brain causes abnormal oxidation of tau and leads to neurodegeneration. Moreover, p-tau is also detected in CSF, and it is known that the amount of p-tau in CSF is frequently correlated with the state of AD.

Neurodegeneration accompanying an increase in excess p-tau has been observed not only in AD, but also in mild cognitive impairment (MCI), frontotemporal dementia, Pick's disease, progressive supranuclear palsy, cortical degeneration, and the like. Such diseases are collectively referred to as "tauopathy".

Meanwhile, conventionally, basil and opal basil, which are herb crops, have been mainly used as cosmetic materials. However, to date, there has been no research on inhibitory activity against tauopathy of basil or opal basil.

### [Prior Art Document]

### [Patent Document]

(Patent Document 1) Korean Patent No. 10-1818736,
GRADINARIU VERONICA ET AL: "Comparative efficacy ofOcimum sanctum L.andOcimum basilicum L.essential oils against amyloid beta (1-42)-induced anxiety and depression in laboratory rats" PHYTOCHEMISTRY REVIEWS, KLUWER, NL, vol. 14, no. 4, 28 November 2014 (2014-11-28), pages 567-575, XP035521514: D1 reveals that essential oils of Ocimum sanctum L. and Ocimum basilicum L. (extracted by hydrodistillation of dried plant material at the early blooming stage) reduced symptoms of anxiety and depression in a rat model of Alzheimer's disease (AD) treated with beta-amyloid.

The above information disclosed in this Background section is only for enhancement of understanding of the background of the invention, and therefore it may contain information that does not form the prior art that is already known in this country to a person of ordinary skill in the art.

### SUMMARY OF THE DISCLOSURE

While searching natural products for a composition for preventing and treating tauopathy or amyloidosis, the present inventors found that a basil extract has the potential to be used in the treatment, prevention or amelioration of Alzheimer's disease through inhibition of aggregation of beta-amyloid proteins and degradation of aggregates thereof or inhibition of aggregation of tau proteins and degradation of aggregates thereof. Based on this finding, the present invention has been completed.

It is one object of the present invention to provide a composition for preventing, ameliorating, or treating Alzheimer's dementia, probable Alzheimer's dementia, possible Alzheimer's dementia, prodromal Alzheimer's dementia, or mild cognitive impairment due to Alzheimer's dementia (MCI due to AD) containing a basil extract as an active ingredient.

It is another object of the present invention to provide a pharmaceutical composition for preventing, ameliorating, or treating Alzheimer's dementia, probable Alzheimer's dementia, possible Alzheimer's dementia, prodromal Alzheimer's dementia, or mild cognitive impairment due to Alzheimer's dementia (MCI due to AD).

It is another object of the present invention to provide a health functional food composition for preventing, ameliorating, or treating Alzheimer's dementia, probable Alzheimer's dementia, possible Alzheimer's dementia, prodromal Alzheimer's dementia, or mild cognitive impairment due to Alzheimer's dementia (MCI due to AD).

In an attempt to achieve the objects described above, the present invention provides the following composition. Further disclosed is a method of preparing the same.

In one aspect, the present invention provides a composition for preventing, ameliorating, or treating tauopathy containing a basil extract as an active ingredient.

The active ingredient acts to inhibit aggregation of tau proteins or degrade tau protein aggregates.

The tauopathy is Alzheimer's dementia, probable Alzheimer's dementia, possible Alzheimer's dementia, prodromal Alzheimer's dementia, or mild cognitive impairment due to Alzheimer's dementia (MCI due to AD.

The basil extract is an extract of water, a lower alcohol having 1 to 5 carbon atoms, or an aqueous solution of a lower alcohol having 1 to 5 carbon atoms.

The basil extract may be an ethanol aqueous solution extract at a concentration of 20% to 80%.

The basil may be sweet basil *(Ocimum basilicum.* L) or opal basil *(Ocimum basilicum* var*. purpureum*).

The basil may be cultivated 65 to 85 days after sowing.

The basil may be germinated at 18 to 23°C under a photoperiod condition of light for 14 hours and dark for 10 hours for 25 to 35 days after sowing.

The basil may be germinated and cultivated in seedling soil having a volume density of 0.1 to 0.5 Mg/m³, a pH of 5 to 7, and an electrical conductance (EC) of 1.2 ds/m or less.

The composition may be a pharmaceutical composition.

The composition may be a health functional food composition.

Further described is a method for preparing the composition, the method including (a) germinating basil, (b) seedling and cultivating the germinated basil, and (c) extracting the cultivated basil.

The basil may be sweet basil (*Ocimum basilicum.* L) or opal basil (*Ocimum basilicum* var. *purpureum*), and the basil in step (a) and/or (b) may be germinated and cultivated in seedling soil having a volume density of 0.1 to 0.5 Mg/m³, a pH of 5 to 7, and an electrical conductance (EC) of 1.2 ds/m or less.

Step (a) may include germinating the basil at 18 to 23°C under a photoperiod condition of light for 14 hours and dark for 10 hours for 25 to 35 days after sowing.

Step (b) may include cultivating the basil at 18 to 25°C 65 to 85 days after sowing.

Step (c) may include extraction after freeze-drying.

Step (c) may include extraction using an aqueous ethanol solution having a concentration of 20% to 80% as an extraction solvent.

The extraction in step (c) may be ultrasonic extraction.

Other aspects and preferred embodiments of the invention are discussed infra.

### BRIEF DESCRIPTION OF THE DRAWINGS

The above and other features of the present invention will now be described in detail with reference to certain exemplary embodiments thereof, illustrated in the accompanying drawings which are given hereinbelow by way of illustration only, and thus are not limitative of the present invention, and wherein:
FIG. 1A shows the yield as a function of cultivation period of basil in Example 1 of the present invention, and FIG. 1B shows the yield as a function of cultivation period of opal basil;
FIG. 2 shows the result of Experimental Example 1, more specifically, FIG. 2A shows the change in the content of functional components of basil, and FIG. 2B shows the change in the content of functional components of opal basil;
FIG. 3 shows the change in antioxidant activity as a function of cultivation period of Experimental Example 2;
FIG. 4 shows the result of Experimental Example 3, more specifically, FIG. 4A showing the result for basil and FIG. 4B showing the result for opal basil;
FIG. 5 shows the result of Experimental Example 4, more specifically, FIG. 5A showing the result for basil and FIG. 5B showing the result for opal basil;
FIGS. 6 and 7 show the result of Experimental Example 5, more specifically, FIG. 6 showing the result for basil and FIG. 7 showing the result for opal basil; and
FIGS. 8 and 9 show the result of Experimental Example 6, more specifically, FIG. 8 showing the result for basil and FIG. 9 showing the result for opal basil.

### DETAILED DESCRIPTION

The objects described above, as well as other objects, features, and advantages, will be clearly understood from the following preferred embodiments with reference to the attached drawings.

Unless the context clearly indicates otherwise, all numbers, figures, and/or expressions that represent ingredients, reaction conditions, polymer compositions, and amounts of mixtures used in the specification are approximations that reflect various uncertainties of measurement occurring inherently in obtaining these figures, among other things. For this reason, it should be understood that, in all cases, the term "about" should be understood to modify all such numbers, figures, and/or expressions. In addition, when numerical ranges are disclosed in the description, these ranges are continuous, and include all numbers from the minimum to the maximum, including the maximum within each range, unless otherwise defined. Furthermore, when the range refers to an integer, it includes all integers from the minimum to the maximum, including the maximum within the range, unless otherwise defined.

Hereinafter, the present invention will be described in detail.

The incidence of Alzheimer's disease increases with age. 3 out of 4 dementia patients over 65 years of age suffer from Alzheimer's dementia. As of 2015, the prevalence of dementia in all people over the age of 65 was 9.8% (about 648,000), and the number of dementia patients is expected to exceed 1 million in 2024. Accordingly, the importance of and interest in ameliorating Alzheimer's disease through food is increasing. As an alternative to the use of drugs to treat the side effects of Alzheimer's disease, prevention thereof through food consumption has been proposed. For example, in 2012, a well-known academic journal, Science, published an article discussing food consumption as a way to prevent cognitive decline and introducing foods for preventing Alzheimer's disease.

Currently, there are a total of five health functional food ingredients certified by the Ministry of Food and Drug Safety in Korea to improve cognitive ability: Angelica root extract, phosphatidylserine, *Lactobacillus Helveticus* fermented products, bellflower extract, and Angelica root extract powder.

The representative etiological factors of Alzheimer's disease known to date are beta-amyloid and tau. To date, there has been much debate about which of the two is more closely related to the disease. However, it is clear that abnormal aggregation of beta-amyloid and tau is found in the brains of Alzheimer's patients, and it is known at present that aggregates of beta-amyloid or tau induce the destruction of brain cells, leading to cognitive impairment.

Therefore, a number of global pharmaceutical companies are developing therapeutic candidates targeting beta-amyloid or tau. Also, most currently commercially available drugs merely temporarily ameliorate cognitive function disorders, and most drug candidates in clinical trials are substances that separately target beta-amyloid or tau. Therefore, there is no substance that controls both beta-amyloid and tau aggregates.

In addition, a key solution to the aggregation of beta-amyloid or tau is not only to inhibit the process of aggregation, but also to remove aggregates that have already formed. A major feature of Alzheimer's disease is that beta-amyloid and tau begin to aggregate long before cognitive impairment appears. When a patient visits a hospital and is diagnosed with Alzheimer's, large amounts of beta-amyloid and tau aggregates are formed. In addition, a normal person has beta-amyloid and tau as monomers. Therefore, an Alzheimer's patient can be distinguished from a normal person based on the presence or absence of aggregates of beta-amyloid and tau. Therefore, inhibiting the aggregation of beta-amyloid and tau and degrading already formed aggregates are very important goals for the development of therapeutic agents for Alzheimer's disease.

In many cases, materials that have been conventionally studied employ only one of the above four mechanisms. For example, one study discloses only the results of research on inhibition of aggregation of beta-amyloid. That is, it discloses only mediation of a beta-amyloid aggregation process to block the progress of aggregation.

Therefore, the researchers of the present invention completed the present invention by extracting, from natural products, ingredients that have effects of inhibiting the aggregation of beta-amyloid and degrading the formed beta-amyloid aggregates, and at the same time, inhibiting the aggregation of tau and degrading the formed tau aggregates. The present inventors found that a basil extract, especially a basil extract obtained under specific germination, cultivation, or extraction conditions, has effects of inhibiting the aggregation of beta-amyloid and degrading previously formed beta-amyloid aggregates, and at the same time, of inhibiting the aggregation of tau and degrading previously formed tau aggregates.

The present inventors identified the potential availability of the basil extract as a health functional food material for ameliorating Alzheimer's disease and conducted repeated experiments based thereon. As a result, the present invention provides a composition for preventing, ameliorating, or treating Alzheimer's disease containing a basil extract as an active ingredient. The present invention can be used as a substitute for a conventional therapeutic agent, the mechanism of which is to provide temporary treatment and alleviation through inhibition and/or degradation of the underlying pathological protein of Alzheimer's disease.

Hereinafter, various aspects of the present invention will be described.

In one aspect, the present invention provides a composition for preventing, ameliorating, or treating Alzheimer's dementia, probable Alzheimer's dementia, possible Alzheimer's dementia, prodromal Alzheimer's dementia, or mild cognitive impairment due to Alzheimer's dementia (MCI due to AD) containing a basil extract as an active ingredient.

As used herein, the term "basil" refers to an annual plant that belongs to the family Lamiaceae. In general, sweet basil is the most common thereof, and there are many different types of basil, such as sweet basil, holy basil, Marseille basil, opal basil, lemon basil, Genovese basil, mammoth basil, cinnamon basil, Christmas basil, anise basil, and purple ruffle basil. In one embodiment, sweet basil or opal basil may be used.

The active ingredient acts to reduce the amount of tau proteins.

The tauopathy is Alzheimer's dementia, probable Alzheimer's dementia, possible Alzheimer's dementia, prodromal Alzheimer's dementia, or mild cognitive impairment due to Alzheimer's dementia (MCI due to AD).

The basil extract is an extract of water, a lower alcohol having 1 to 5 carbon atoms, or an aqueous solution of a lower alcohol having 1 to 5 carbon atoms.

The basil extract is an ethanol aqueous solution extract at a concentration of 20% to 80%. In one embodiment, the basil extract is an ethanol aqueous solution extract at a concentration of 30% to 50%. When the extraction conditions are satisfied, the therapeutic effect of tauopathy is excellent.

The basil is sweet basil (*Ocimum basilicum.* L) or opal basil (*Ocimum basilicum* var. *purpureum*).

The basil is cultivated 65 to 85 days after sowing. When the requirement for cultivation after sowing is satisfied, the therapeutic effect on tauopathy is excellent.

The basil is germinated at 18 to 23°C under a photoperiod condition of 14 hours/10 hours for 25 to 35 days after sowing. When the requirement for germination after sowing is satisfied, the therapeutic effect on tauopathy is excellent.

In one embodiment, the 14 hours of the photoperiod condition is considered daytime, and the remaining 10 hours is considered nighttime.

The basil is germinated and cultivated in seedling soil having a volume density of 0.1 to 0.5 Mg/m³, a pH of 5 to 7, and an electrical conductance (EC) of 1.2 ds/m or less. When the requirement for germination or cultivation is satisfied, the therapeutic effect on tauopathy is excellent.

The term "electrical conductance (EC)" refers to the concentration of ions dissolved in a culture medium. A high EC indicates a large amount of ions and a high nutrient concentration.

The composition may be a pharmaceutical composition.

Carriers, excipients and diluents that may be contained in the pharmaceutical composition of the present invention include one or more selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, hydroxymethyl cellulose, microcrystalline cellulose, silicified microcrystalline cellulose, povidone, crospovidone, croscarmellose sodium, polyvinylpyrrolidone, water, methylhydroxybenzoate, propylhydroxybenzoate, Noisirin, colloidal silicon dioxide, lactose, talc, magnesium stearate, colloidal magnesium stearate, and mineral oil.

The amount of the pharmaceutical composition of the present invention that is administered may be determined by those skilled in the art in consideration of other well-known factors in the medical field, such as the purpose of use, the severity of disease, age, weight, state of health, gender, sensitivity to drugs, administration time, administration route, or the type of substance used as an active ingredient. The composition may contain a basil extract as a single active ingredient. That is, the composition may not contain an active ingredient other than the basil extract. In one embodiment, there is provided a method of treating Alzheimer's dementia, probable Alzheimer's dementia, possible Alzheimer's dementia, prodromal Alzheimer's dementia, or mild cognitive impairment due to Alzheimer's dementia (MCI due to AD) including administering to a subject the pharmaceutical composition in an amount effective to prevent or treat the Alzheimer's dementia, probable Alzheimer's dementia, possible Alzheimer's dementia, prodromal Alzheimer's dementia, or mild cognitive impairment due to Alzheimer's dementia (MCI due to AD). The subject may be a mammal. The mammal may be a human, dog, cat, cow, goat, or pig. In addition, the subject may be a human or non-human animal, and may be a human or non-human mammal. Administration may be performed as desired through any general route, as long as the composition can reach the target tissue, for example, through application to the skin, intraperitoneal administration, intravenous administration, intramuscular administration, subcutaneous administration, intradermal administration, transdermal patch application, oral administration, intranasal administration, intrapulmonary administration, rectal administration, topical administration, eye drop administration, and the like, specifically through eye drop administration or the like. The preferred dosage of the pharmaceutical composition of the present invention varies depending on the patient's age and weight, disease severity, drug form, and administration route and period, but may be appropriately selected by those skilled in the art. However, for desirable effects, the pharmaceutical composition of the present invention may be administered in an amount of 0.001 mg/kg to 1 mg/kg per day, preferably 0.1 mg/kg to 10 mg/kg per day. Administration may be performed several times a day, preferably divided into 1 to 6 doses, at regular time intervals according to the prescription of a doctor or pharmacist.

The composition for preventing, ameliorating or treating Alzheimer's dementia, probable Alzheimer's dementia, possible Alzheimer's dementia, prodromal Alzheimer's dementia, or mild cognitive impairment due to Alzheimer's dementia (MCI due to AD) may be a health functional food composition.

The health food composition of the present invention contains a basil extract, and there is no particular limitation as to the type thereof. Examples of the food include drinks, meats, sausages, bread, biscuits, rice cakes, Sunsik (Korean ready-to-eat food prepared from grains), chocolate, candy, snacks, confectioneries, pizza, ramen, other noodles, gums, dairy products including ice cream, various soups, beverages, alcoholic beverages, vitamin complexes, dairy products, and processed dairy products, and include all other functional health foods in the conventional sense. As an active ingredient, a basil extract powder or dried powder thereof may be added alone to the food, or may be used in conjunction with other foods or food ingredients, and may be suitably used according to conventional methods. The effective content may be appropriately determined according to the purpose of use (for prevention or amelioration), and may be within a range of 0.001 to 70% by weight with respect to the total weight of the health food. However, in the case of long-term intake for health and hygiene purposes or for health control, the amount may be below the above range, and the active ingredient may be also used in an amount above the range, since there is no problem with regard to safety. For example, in the case of preparing health beverages, the health drink may contain, in addition to the active ingredient, natural carbohydrates or flavoring agents, which are additives commonly used in the preparation of beverages. The natural carbohydrates may include conventional sugars, such as monosaccharides (e.g. glucose, fructose, etc.), disaccharides (e.g. maltose, sucrose, etc.), and polysaccharides (e.g., dextrin, cyclodextrin, etc.), and sugar alcohols such as xylitol, sorbitol, and erythritol. The natural carbohydrate may be present in a range of 1 to 20% by weight, preferably 5 to 10% by weight, with respect to the total weight of the health food. The flavoring agent may include natural flavoring agents (thaumatin, stevia extract, rebaudioside A, glycyrrhizin, etc.) and synthetic flavoring agents (saccharin, aspartame, etc.). The health food may contain other nutrients, vitamins, minerals (electrolytes), flavors (synthetic or natural flavors), colorants, pectic acids and salts thereof, alginic acids and salts thereof, organic acids, protective colloidal thickeners, pH-adjusting agents, stabilizers, preservatives, glycerin, alcohol, carbonic acid used in carbonated beverages, and the like. In addition, it may contain flesh for the production of natural fruit juices, fruit juice beverages, and vegetable beverages. The content of these additives is not particularly limited, but may fall within a range of 0.1 to 20% by weight with respect to the total weight of the health food.

The active ingredient contained in the composition according to one or another aspect of the present invention may be present in an amount of 0.01% to 50% by weight based on the total weight of the composition.

Further described is a method for preparing the composition for ameliorating, preventing or treating Alzheimer's dementia, probable Alzheimer's dementia, possible Alzheimer's dementia, prodromal Alzheimer's dementia, or mild cognitive impairment due to Alzheimer's dementia (MCI due to AD) of the present invention, the method including: (a) germinating basil; (b) seedling and cultivating the germinated basil; and (c) extracting the cultivated basil.

The basil is sweet basil (*Ocimum basilicum.* L) or opal basil (*Ocimum basilicum* var*. purpureum*) and the basil in step (a) and/or (b) is germinated and cultivated in seedling soil having a volume density of 0.1 to 0.5 Mg/m³, a pH of 5 to 7, and an electrical conductance (EC) of 1.2 ds/m or less.

Step (a) includes germinating the basil at 18 to 23°C under a condition of light for 14 hours, followed by dark for 10 hours for 25 to 35 days after sowing.

Step (b) includes cultivating the basil at 18 to 25°C 65 to 85 days after sowing.

Step (c) includes extraction after freeze-drying.

Step (c) includes extraction using an aqueous ethanol solution having a concentration of 20% to 80% as an extraction solvent.

The extraction in step (c) is ultrasonic extraction.

Hereinafter, the present invention will be described in more detail with reference to specific examples. However, the following examples are provided only for better understanding of the present invention, and thus should not be construed as limiting the scope of the present invention.

### Example 1. Determination of optimal cultivation time

### 1-1. Cultivation conditions

Sweet basil (*Ocimum basilicum* L.) and opal basil (*Ocimum basilicum* var*.* purpureum)
seeds commercially available from Danone were prepared.

Seeds of two varieties were germinated in seedling soil (volume density = 0.3 Mg/m³; pH = 5-7; EC ≤ 1.2 ds/m) for 30 days at a temperature of 18 to 23°C under a photoperiod condition of 14 h / 10 h.

A month later, cultivation was performed at a temperature of 18 to 25°C and harvesting was performed on days 30, 60, 70 and 80 based on the sowing date.

### 1-2. Determination of yield

The yield was determined in units of grams per plant by cultivating and harvesting each variety and then measuring the weight of each plant.

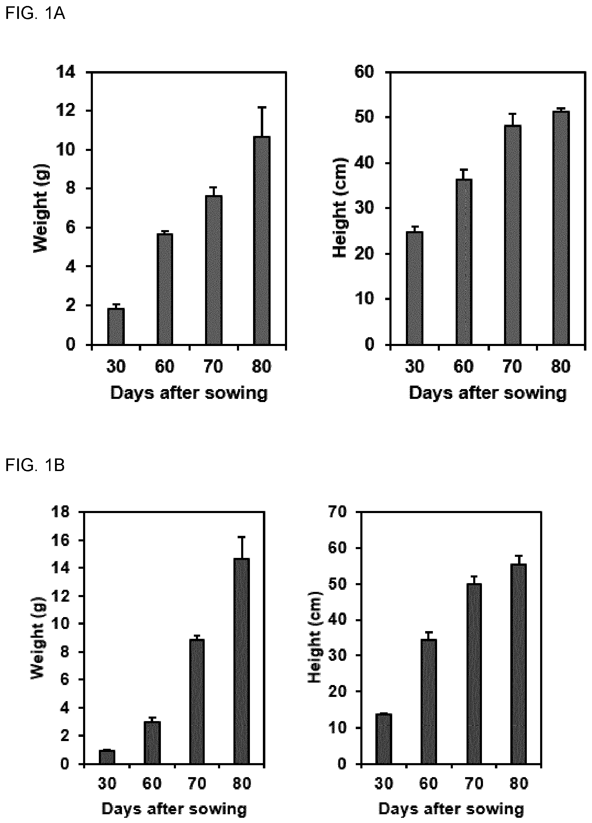

The yield as a function of the cultivation period is shown in FIGS. 1A and 1B below.

### Example 2. Preparation of basil extract

The raw materials of various basil samples germinated and cultivated in Example 1 were extracted under the following conditions.

Plants were freeze-dried at -70°C and extracted using ultrasonic extraction with 40% ethanol (5 ml/1g). The extract was concentrated with a nitrogen concentrator and dissolved again in DMSO at a concentration of 40 mg/ml for subsequent experiments.

### Experimental Example 1. Changes in content of functional ingredients as function of cultivation period

The basil extracts obtained in Examples 1 and 2 were used in the following experiments.

The total phenolic content was analyzed by the following method.

200 µL of a 2% sodium carbonate solution was added to 10 µL of the sample, followed by reaction for 3 minutes. Then, 10 µL of a 1N Folin solution was added to the mixture and reacted in the dark for 27 minutes and absorbance was measured. A calibration curve was drawn using gallic acid as a standard, and the results were expressed as mgGAE/g.

The rosmarinic acid content was analyzed through the following method.

10 µL of a sample was injected into an Agilent 1200 series liquid chromatography (HPLC) apparatus, an ODS-II C18 column from Supersil was used as an analytical column, and the temperature of the column was maintained at 40°C during the analysis. Distilled water (A) containing 0.2% formic acid and acetonitrile (B) containing 0.2% formic acid were used as solvents, the solvent flow rate was set to 1 ml/min, and the ratio of solvent B was changed to 10% for 0-1 minute, to 50% for 15 minutes, to 100% for 25 minutes, and to 10% for 26 minutes.

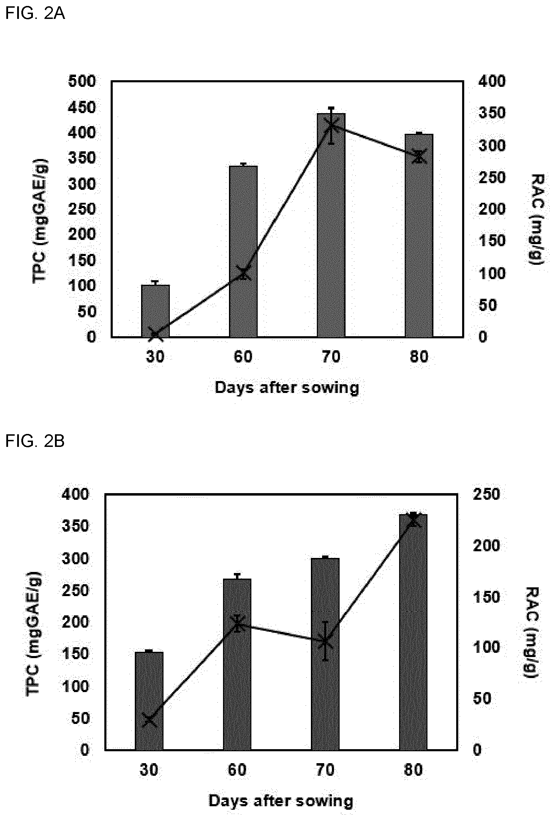

The experimental results are shown in FIG. 2 below. FIG. 2A shows basil and FIG. 2B shows opal basil.

### Experimental Example 2. Changes in antioxidant activity as function of cultivation period

The basil extracts obtained in Examples 1 and 2 were used in the following experiments.

An ABTS radical scavenging assay to evaluate antioxidant activity was performed as follows. 90 µL of an ABTS solution activated with 2.45 mM potassium persulfate was added to 10 µL of the sample and reacted at room temperature in the dark for 10 minutes. The absorbance of the reaction product was measured through UV-VIS spectrometry.

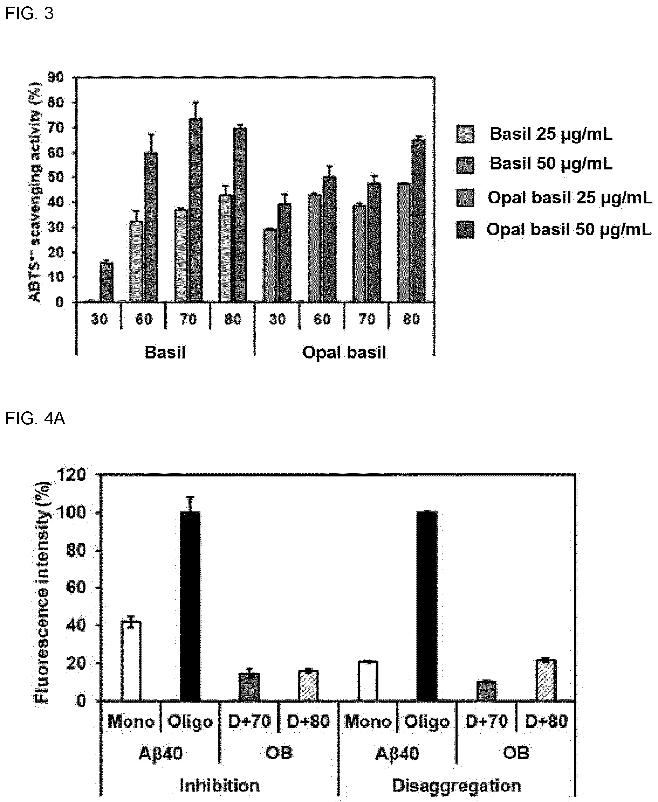

The experimental results were as shown in FIG. 3.

### Experimental Example 3. Alzheimer's disease inhibitory activity - effects of inhibiting beta-amyloid aggregation and degradation of beta-amyloid aggregates

The basil extracts obtained in Examples 1 and 2 were used in the following experiments.

In order to analyze the inhibitory activity against Alzheimer's disease, the effect of inhibiting the aggregation of beta-amyloid, which is a representative etiological factor of Alzheimer's disease, or degrading the formed beta-amyloid aggregates was evaluated through the following method. In order to analyze beta-amyloid aggregation inhibitory activity, 100 µl of synthetic beta-amyloid (Aβ40) at a concentration of 20 µM was stored in an e-tube at -80°C to prevent aggregation to form a monomer. At the same time, to form an oligomer, the same amount and concentration of synthetic beta-amyloid (Aβ40) was fed to a black 96-well plate and incubated at 37°C for 5 days. In addition, a mixture of a basil extract (40 µg/ml) and beta-amyloid (Aβ40) was added to the same plate and incubated at 37°C for 5 days. During this period, the plate was lightly tapped daily to prevent self-aggregation of the basil extract and beta-amyloid. After 5 days, PBS and the monomer used as blanks were placed on the same plate in the same way, and then Thioflavin-T (ThT) analysis was performed. Th-T analysis was performed by adding 75 µl of 5 µM Th-T dissolved in glycine buffer (pH 8.9, 50 mM) to each well containing the basil extract or beta-amyloid. Then, the fluorescence intensity was measured at an excitation wavelength of 450 nm and an emission wavelength of 485 nm for 3 seconds during shaking.

To analyze the degradation efficacy of previously formed beta-amyloid aggregates, the monomer was stored in the same manner as above, and the polymer incubated in an e-tube at 37°C for 5 days in advance was diluted to 500 µM. The final beta-amyloid (Aβ40) concentration measured in the experiment was 20 µM. The concentration of the basil extract mixed with the formed 500 µM beta-amyloid (Aβ40) polymer was 40 µg/ml. The remainder of the experimental procedure was performed in the same manner as above.

The experimental results are shown in FIG. 4 below. FIG. 4A shows the results for basil and FIG. 4b shows the results for opal basil.

### Experimental Example 4. Alzheimer's disease inhibitory activity - effects of inhibiting tau aggregation and degradation of tau aggregates

The basil extracts obtained in Examples 1 and 2 were used in the following experiments.

In order to analyze the inhibitory activity against Alzheimer's disease, the effect of inhibiting the aggregation of tau, which is another representative etiological factor of Alzheimer's disease, or of degrading formed tau aggregates was evaluated through the following method. 0.1 mg/ml of heparin and 10 µM of dithiothreitol (DTT) were mixed with tau at a final tau concentration of 0.5 mg/ml to form tau aggregates. The experiment was performed in the same manner as in Experimental Example 3, except that beta amyloid (Aβ40) was replaced with tau.

To analyze the effect of degrading tau aggregates, tau monomers were stored in the same manner as beta-amyloid in Experimental Example 3. In addition, 1 mg/ml of the previously formed tau polymer was diluted to 0.5 mg/ml and used. The experiment was performed in the same manner as Experimental Example 3.

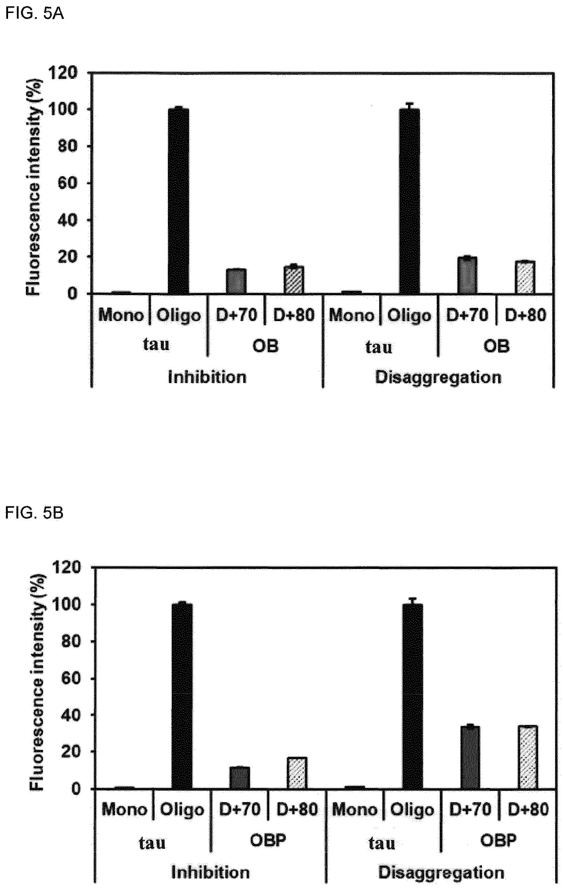

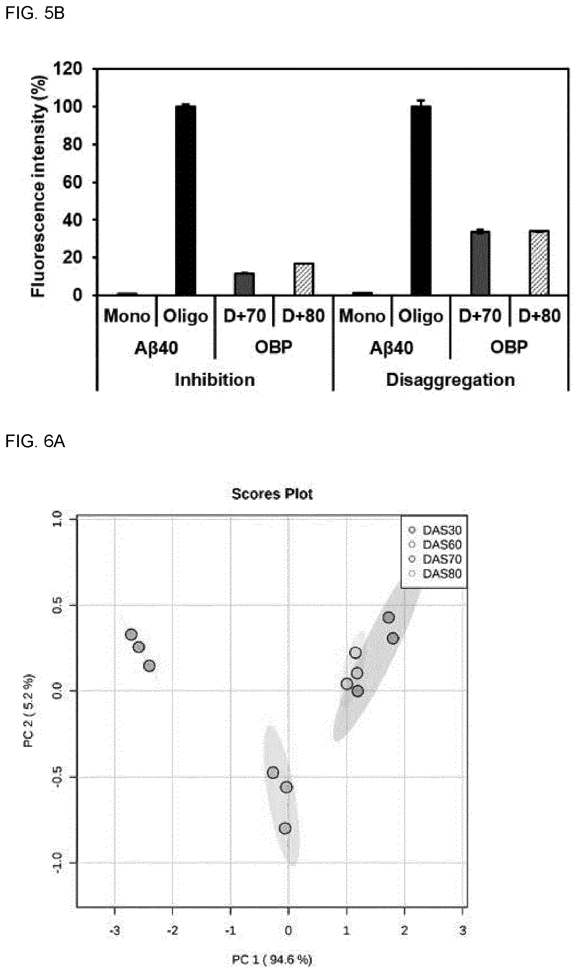

The experimental results are shown in FIG. 5 below. FIG. 5A shows basil and FIG. 5B shows opal basil.

### Experimental Example 5. Correlation analysis of functional ingredients and physiological activity

A score plot for analyzing the relationship of the plant with the number of days after sowing was obtained using the SIMCA program, and the correlation between functional ingredients and physiological activity was analyzed using MetaboAnalyst.

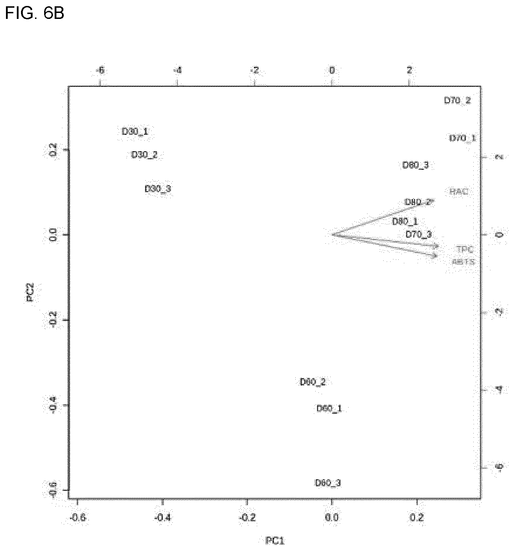

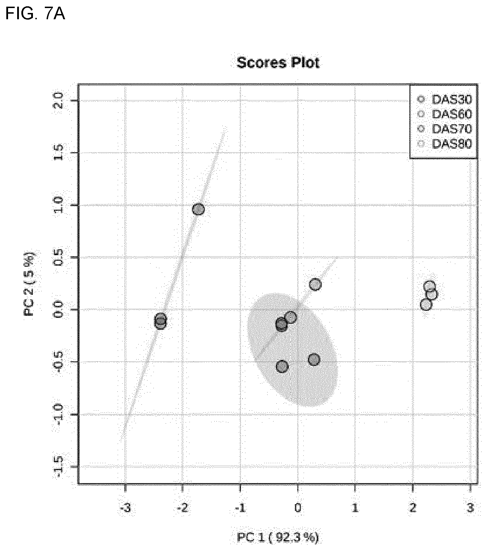

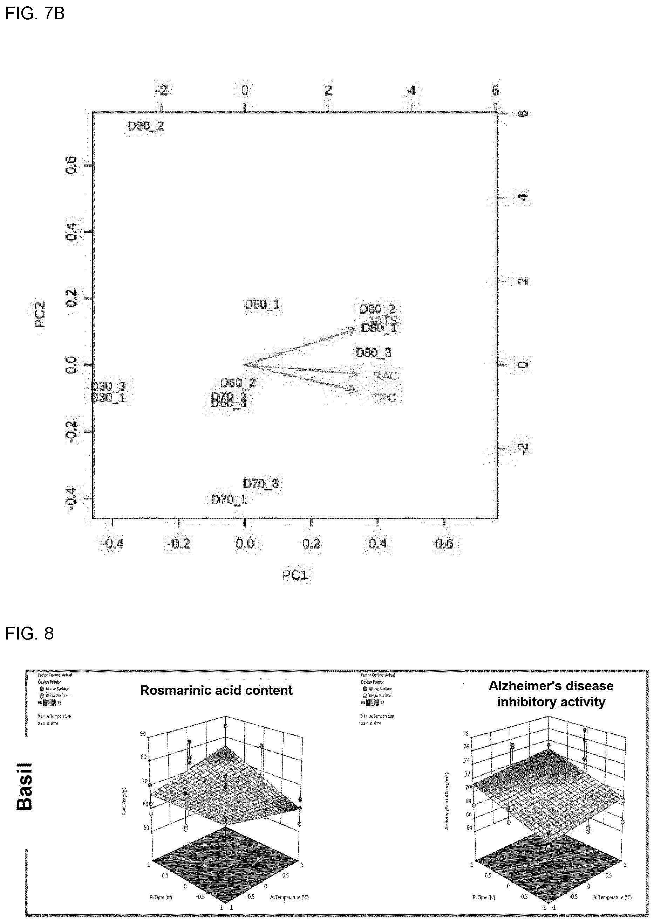

The experimental results are shown in FIGS. 6 and 7 below. FIG. 6 shows the results for basil and FIG. 7 shows the results for opal basil.

### Experimental Example 6. Analysis of optimal extraction conditions for functional ingredients

Response surface analysis to determine optimal extraction conditions was performed using Design-expert software. The content of rosmarinic acid, a functional ingredient, and Alzheimer's inhibitory activity under respective conditions were used as data.

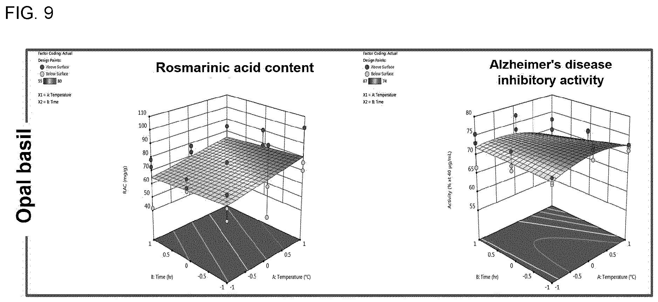

The experimental results are shown in FIGS. 8 and 9 below.

Hereinafter, formulation examples according to the present invention will be described in more detail. However, the following formulation examples are provided only for better understanding of the present invention, and thus should not be construed as limiting the scope of the present invention.

### [Formulation Example 1] Preparation of pill

30% by weight of the basil extract of Example 2, 30% by weight of corn starch, 20% by weight of glycerin, and 20% by weight of a sorbitol powder were mixed, and a pill was produced using a pill-making machine. The final weight of the product was 3.5 g.

### [Formulation Example 2] Preparation of tablet

30% by weight of the basil extract of Example 2, 20.5% by weight of lactose, 20% by weight of dextrin, 20% by weight of a maltitol powder and 7% by weight of a xylitol powder were mixed, granulated using a fluidized bed dryer, added with 2.5% sugar ester by weight, and then tableted with a tableting machine. The final weight of the product was 2 g.

### [Formulation Example 3] Preparation of granules

30% by weight of the basil extract of Example 2, 5% by weight of xylitol, and 65% by weight of isomalt were mixed, formed into granules using a fluidized bed granulator, and then placed in a bag. The final weight of the product was 2 g.

### [Formulation Example 4] Health food

A health food having the composition shown in Table 1 below was prepared in a conventional manner.

**[Table 1]**

| Ingredient | Content |
|---|---|
| Example 2 | 20 mg |
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| Nicotinic acid amide | 1.7 mg |
| Folic acid | 50 µg |
| Calcium pantothenate | 0.5 mg |
| Ferrous sulfate | 1.75 mg |
| Zinc oxide | 0.82 mg |
| Magnesium carbonate | 25.3 mg |
| Monobasic potassium phosphate | 15 mg |
| Dicalcium phosphate | 55 mg |
| Potassium citrate | 90 mg |
| Calcium carbonate | 100 mg |
| Magnesium chloride | 24.8 mg |

### [Formulation Example 5] Health beverage

A health beverage having the composition shown in Table 2 below was prepared in a conventional manner.

**[Table 2]**

| Ingredient | Content |
|---|---|
| Example 2 | 1000 mg |
| Citric acid | 1000 mg |
| Oligosaccharide | 100 g |
| Taurine | 1 g |
| Purified water | Balance |

As is apparent from the foregoing, the composition according to one aspect of the present invention can be used to prevent, ameliorate, or treat Alzheimer's dementia, probable Alzheimer's dementia, possible Alzheimer's dementia, prodromal Alzheimer's dementia, or mild cognitive impairment due to Alzheimer's dementia (MCI due to AD).

The composition according to one aspect of the present invention can be used to prevent, ameliorate, or treat Alzheimer's dementia, probable Alzheimer's dementia, possible Alzheimer's dementia, prodromal Alzheimer's dementia, or mild cognitive impairment due to Alzheimer's dementia (MCI due to AD) through inhibition of the aggregation of tau proteins.

The composition according to one aspect of the present invention can be used to prevent, ameliorate, or treat Alzheimer's dementia, probable Alzheimer's dementia, possible Alzheimer's dementia, prodromal Alzheimer's dementia, or mild cognitive impairment due to Alzheimer's dementia (MCI due to AD) through degradation of the aggregates of tau proteins.

The composition has effects of inhibiting beta-amyloid aggregation, of degrading formed beta-amyloid aggregates, of inhibiting tau aggregation, and of degrading formed tau aggregates.

The composition exhibits excellent antioxidant activity.

The composition is a pharmaceutical composition or a health functional food composition effective for tauopathy including Alzheimer's disease

The present invention has been described in detail with reference to embodiments thereof.

## Claims

1. A basil extract as an active ingredient for use in preventing, ameliorating or treating Alzheimer's dementia, probable Alzheimer's dementia, possible Alzheimer's dementia, prodromal Alzheimer's dementia, or mild cognitive impairment due to Alzheimer's dementia (MCI due to AD), wherein the active ingredient acts to inhibit aggregation of tau proteins or/and degrade tau protein aggregates, wherein the basil extract is an extract of water, a lower alcohol having 1 to 5 carbon atoms, or an aqueous solution of a lower alcohol having 1 to 5 carbon atoms, or
wherein the basil extract is an ethanol aqueous solution extract at a concentration of 20% to 80%.

2. The basil extract according to claim 1, wherein the basil is sweet basil *(Ocimum basilicum.* L) or opal basil *(Ocimum basilicum* var. *purpureum*), and optionally wherein the basil is cultivated 65 to 85 days after sowing.

3. The basil extract according to one of claims 1 and 2, wherein the basil is germinated at 18 to 23°C under a photoperiod condition of light for 14 hours and dark for 10 hours for 25 to 35 days after sowing.

4. The basil extract according to claim 3, wherein the basil is germinated and cultivated in seedling soil having a volume density of 0.1 to 0.5 mg/m³, a pH of 5 to 7, and an electrical conductance (EC) of 1.2 ds/m or less.

5. The basil extract according to one of claims 1 to 4, wherein the basil extract is administered as form of a pharmaceutical composition.

6. The basil extract according to one of claims 1 to 4, wherein the basil extract is administered as form of a health functional food composition.

## Patentansprüche

1. Basilikumextrakt als Wirkstoff zur Verwendung bei der Vorbeugung, Linderung oder Behandlung von Alzheimer-Demenz, wahrscheinlicher Alzheimer-Demenz, möglicher Alzheimer-Demenz, prodromaler Alzheimer-Demenz oder leichter kognitiver Beeinträchtigung aufgrund von Alzheimer-Demenz, wobei der Wirkstoff durch Hemmung der Anlagerung von Tau-Proteinen oder/und durch Abbau von Tau-Proteinanlagerungen wirkt, wobei der Basilikumextrakt ein Extrakt aus Wasser, einem niedrigwertigen Alkohol mit 1 bis 5 Kohlenstoffatomen oder einer wässrigen Lösung aus einem niedrigwertigen Alkohol mit 1 bis 5 Kohlenstoffatomen ist oder
wobei der Basilikumextrakt ein Extrakt aus wässriger Ethanol-Lösung mit einer Konzentration von 20 % bis 80 % ist.

2. Basilikumextrakt nach Anspruch 1, wobei es sich bei dem Basilikum um grünes Basilikum (*Ocimum basilicum* L.) oder rotes Basilikum (*Ocimum basilicum* var. *purpureum*) handelt und wobei das Basilikum nach dem Säen 65 bis 85 Tage lang kultiviert wird.

3. Basilikumextrakt nach Anspruch 1 oder 2, wobei die Keimung des Basilikums bei 18 bis 23 °C unter Photoperiodenbedingungen von 14 Stunden Licht und
10 Stunden Dunkelheit über eine Dauer von 25 bis 35 Tagen nach dem Säen erfolgt.

4. Basilikumextrakt nach Anspruch 3, wobei die Keimung und Kultivierung des Basilikums in Anzuchterde mit einer Volumendichte von 0,1 bis 0,5 mg/m³, einem pH-Wert von 5 bis 7 und einer elektrischen Leitfähigkeit von 1,2 ds/m oder weniger erfolgt.

5. Basilikumextrakt nach einem der Ansprüche 1 bis 4, wobei der Basilikumextrakt als Form einer pharmazeutischen Zusammensetzung verabreicht wird.

6. Basilikumextrakt nach einem der Ansprüche 1 bis 4, wobei der Basilikumextrakt als Form einer Nutrazeutikum-Zusammensetzung verabreicht wird.

## Revendications

1. Extrait de basilic comme ingrédient actif pour l'utilisation dans la prévention, l'amélioration ou le traitement de la maladie d'Alzheimer, de la maladie d'Alzheimer probable, de la maladie d'Alzheimer possible, de la maladie d'Alzheimer prodromique ou d'un déficit cognitif léger à cause de la maladie d'Alzheimer, dans lequel l'ingrédient actif inhibe l'accumulation de protéines tau ou/et dégrade des accumulations de protéines tau, dans lequel l'extrait de basilic est un extrait d'eau, d'un alcool de faible valeur ayant 1 à 5 atomes de carbone ou d'une solution aqueuse d'un alcool de faible valeur ayant 1 à 5 atomes de carbone ou
dans lequel l'extrait de basilic est un extrait de solution aqueuse d'éthanol à une concentration de 20 % à 80 %.

2. Extrait de basilic selon la revendication 1, dans lequel le basilic est le basilic vert (*Ocimum basilicum* L.) ou le basilic rouge (*Ocimum basilicum* var. *purpureum*), et dans lequel le basilic est cultive optionnellement 65 à 85 jours après l'ensemencement.

3. Extrait de basilic selon la revendication 1 ou 2, dans lequel le basilic est fait germer à 18 à 23 °C sous une condition de photopériode de lumière pour 14 heures et d'obscurité pour 10 heures pendant 25 à 35 jours après l'ensemencement.

4. Extrait de basilic selon la revendication 3, dans lequel le basilic est fait germer et cultivé dans du terreau semis ayant une densité de volume de 0,1 à 0,5 mg/m³, un pH de 5 à 7 et une conductance électrique de 1,2 ds/m ou moins.

5. Extrait de basilic selon l'une quelconque des revendications 1 à 4, dans lequel l'extrait de basilic est administré comme forme d'une composition pharmaceutique.

6. Extrait de basilic selon l'une quelconque des revendications 1 à 4, dans lequel l'extrait de basilic est administré comme forme d'une composition nutraceutique.
